(19) Europäisches Patentamt
European Patent Office
Office européen des brevets

(11) Veröffentlichungsnummer: **0 024 358**
**A1**

(12) # EUROPÄISCHE PATENTANMELDUNG

(21) Anmeldenummer: **80105810.8**

(22) Anmeldetag: **26.07.79**

(51) Int. Cl.³: **C 07 F 7/18**, C 07 C 13/23, C 07 C 35/18 // A61K7/46

(30) Priorität: **10.08.78 CH 8520/78**

(43) Veröffentlichungstag der Anmeldung: **04.03.81 Patentblatt 81/9**

(84) Benannte Vertragsstaaten: **CH DE FR GB NL**

(71) Anmelder: **L. Givaudan & Cie Société Anonyme, Patentdienst Postfach, CH-4002 Basel (CH)**

(72) Erfinder: **Oppolzer, Wolfgang, Prof. Dr., 4b, Chemin de la Cocuaz, CH-1253 Vandoeuvres (CH)**

(74) Vertreter: **Aschert, Hubert et al, Postfach 601 Grenzacherstrasse 124, CH-4002 Basel (CH)**

(54) **Substituierte Cyclohexadiene.**

(57) Die Erfindung betrifft substituierte Cyclohexadiene der Formel

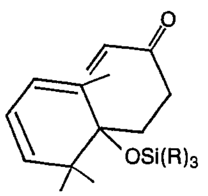

IV

worin R eine niedere Alkylgruppe, gegebenenfalls nieder Alkyl-substituiertes Phenyl oder Phenylniederalkyl bedeutet, worin die niederen Alkylgruppen 1-4 Kohlenstoffatome enthalten.

Diese Verbindungen sind Zwischenprodukte zur Herstellung von Norpatchoulenol.

EP 0 024 358 A1

L. Givaudan & Cie Société Anonyme, Vernier-Genève (Schweiz)

Ref. 6510/171-001

## Substituierte Cyclohexadiene

Die vorliegende Erfindung betrifft substituierte Cyclohexadiene. Diese Verbindungen sind wertvolle Zwischenprodukte zur Herstellung von Norpatchoulenol. (Siehe unsere europäische Anmeldung No. 79.102661.0).

Die substituierten Cyclohexadiene gemäss der vorliegenden Erfindung sind Verbindungen der Formel

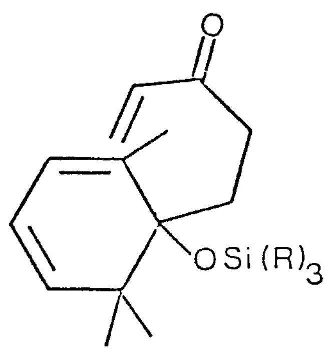

IV

worin R eine niedere Alkylgruppe, gegebenenfalls nieder Alkyl-substituiertes Phenyl oder Phenylniederalkyl bedeutet, worin die niederen Alkylgruppen 1-4 Kohlenstoffatome enthalten.

Die Cyclohexadiene der Formel IV können in an sich bekannter Weise hergestellt werden, beispielsweise durch Behandlung eines 2,2,6-Trimethylcyclohexa-2,4-dienons mit einem 3-trisubstituierten Silyloxydienyl-lithium der Formel

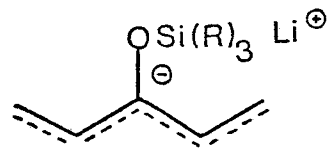

II

worin R die obige Bedeutung hat, und die gestrichelten Linien die Gegenwart von zwei konjugierten Doppelbindungen andeuten,

und darauffolgende Behandlung des erhaltenen Produktes mit einem trisubstituierten Silylhalogenid, vorzugsweise einem Triniederalkylsilylhalogenid, beispielsweise Trimethylsilylchlorid, unter Bildung des Tetraens der Formel

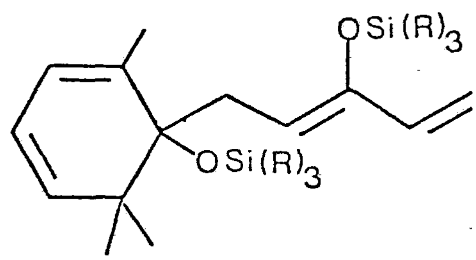

III

Die selektive Desilylierung dieses Tetraens mit Kaliumfluorid in Methanol bei einer Temperatur zwischen etwa 0 und 5°C ergibt nach Chromatographie das gewünschte Cyclohexadiene der Formel

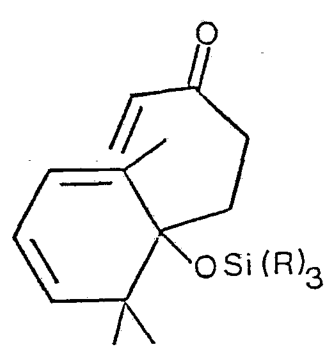

IV

Die Substituenten R können gleich oder voneinander verschieden sein und beispielsweise Methyl, Aethyl, Propyl Butyl, Phenyl, Benzyl oder Tolyl darstellen. Vorzugsweise sind die Substituenten R jeweils gleich und bedeuten Methyl oder Aethyl.

Die verschiedenen Stufen, in welchen die Verbindung der Formel IV in das Norpatchoulenol übergeführt werden kann, sind in unserer europäischen Anmeldung No. 79.102661.0 beschrieben. Der Gesamtverlauf der Synthese von Norpatchoulenol unter Verwendung von Cyclohexadienen der vorliegenden Erfindung, ausgehend von 2,2,6-Trimethylcyclohexa-2,4-dienon, kann durch das folgende Formelschema wiedergegeben werden:

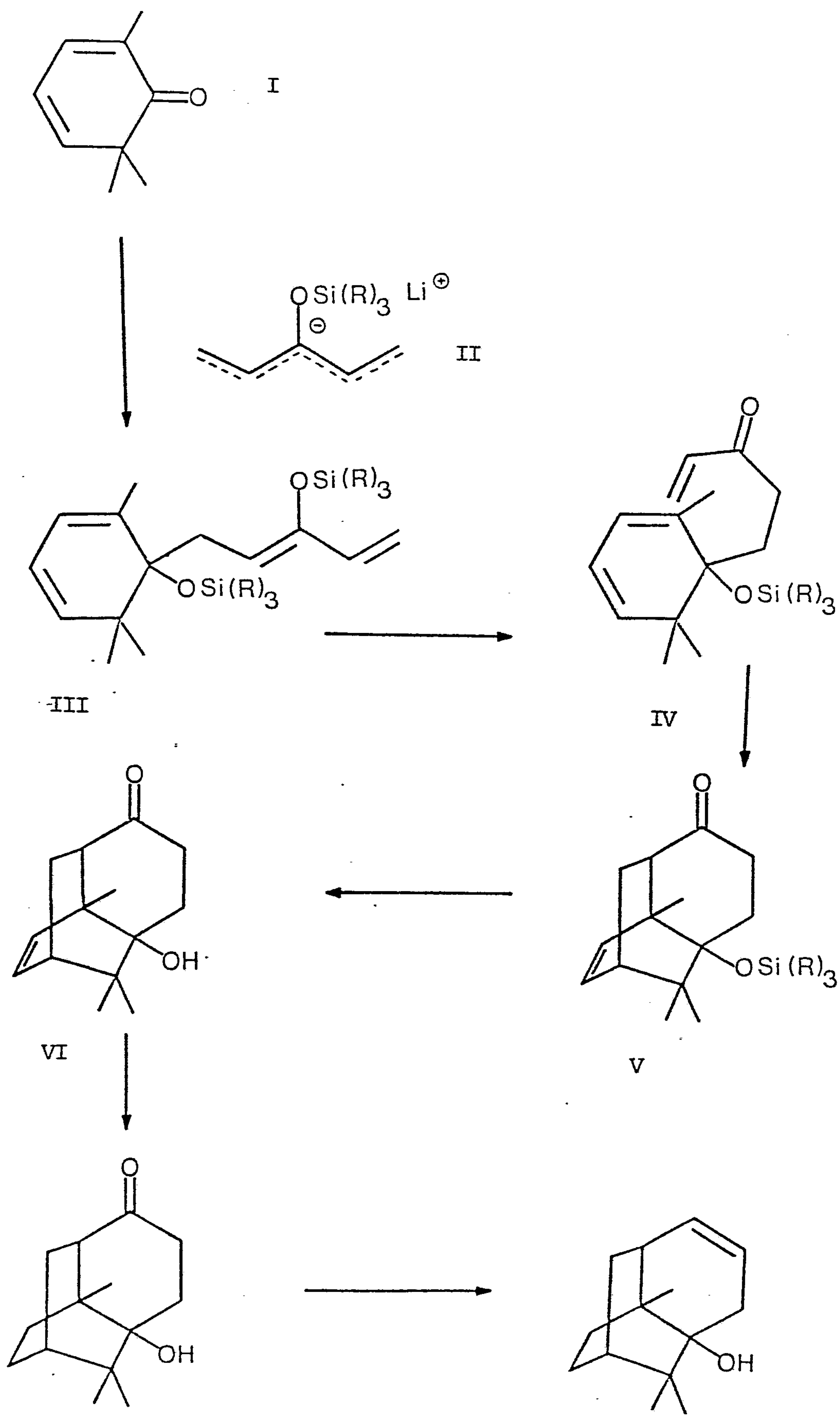

I

II

III

IV

V

VI

VII

VIII

0024358

Die Cyclohexadiene der Formel I ermöglichen es, sowohl (±), als auch (+), als auch (-)-Norpatchoulenol herzustellen, und zwar je nach der Konfiguration der verwendeten Verbindung der Formel I. Bei Verwendung einer optisch aktiven Verbindung erhält man dementsprechend ein optisch aktives Endprodukt. Zur Herstellung des (+)-Norpatchoulenols, wie es in natürlichem Patchouliöl vorkommt, ist eine trisubstituierte Silylverbindung der Formel I zu verwenden, welche in S-Konfiguration vorliegt.

0024358

## Beispiel

Zu einer Lösung von 400 mg 3-Trimethylsilyloxy-1,4-pentadien in 3 ml Tetrahydrofuran unter Argon wurde eine Lösung von 2,3 ml sec-Butyllithium in Cyclohexan und nachher eine Lösung von 275 mg 2,6,6-Trimethyl-cyclohexa-2,4-dienon in 1 ml Tetrahydrofuran zugegeben. Nach 5 Minuten wurde 332 mg Trimethylsilylchlorid in 2 ml Hexamethylphosphorsäuretriamid zugesetzt und das Gemisch eine Stunde bei -78° gelassen und schliesslich auf gesättigte wässrige $NH_4Cl$-Lösung gegossen. Extraktion mit Pentan und Eindampfen der getrockneten Extrakte ergab ein farbloses Oel. Das rohe Produkt wurde mit 20 ml Methanol verdünnt bis 0° gekühlt und 224 mg Kaliumfluorid wurde zugegeben. Das Gemisch wurde 1 Stunde bei 0 bis 5°C gerührt, anschliessend auf gesättigte wässrige $NH_4Cl$-Lösung gegossen und mit Aether extrahiert. Chromatographie der eingedampften Extrakte ($SiO_2$, Toluen) ergab 329 mg 1-[3-Oxo-pent-4-en-1-yl]-trimethylsilyloxy-2,2,6-trimethylcyclohexa-2,4-dien (56% Ausbeute), charakterisiert wie oben.

Das Ausgangsmaterial kann wie folgt hergestellt werden:

Zu einer gerührten Lösung von 272 mg 2,2,6-Trimethyl-cyclohexa-2,4-dienon in 2 ml Tetrahydrofuran wurde eine Lösung von 2 mMol frisch bereitetem 3-Triäthylsilyloxypentadienyl-lithium in 4 ml Tetrahydrofuran bei -78°C zugetropft. Das Reaktionsgemisch wurde 5 Minuten bei -78°C gerührt, dann mit 1 ml Hexamethylphosphorsäuretriamid, darauffolgend mit 0,4 ml Trimethylsilylchlorid (0,4 ml) versetzt, eine weitere Stunde bei -78°C gerührt und schliesslich auf gesättigte wässrige $NH_4Cl$-Lösung gegossen. Extraktion mit Pentan und Eindampfen der getrockneten Extrakte ergab ein farbloses Oel, das in 30 ml Methanol gelöst wurde. Zu dieser Lösung wurden bei 0°C unter Argon und Rühren portionenweise 400 mg Kaliumfluorid zugesetzt. Das Gemisch wurde 1 Stunde bei 0 bis 5°C gerührt, anschliessend auf gesättigte wässrige $NH_4Cl$-Lösung gegossen und mit Aether extrahiert. Chromatographie der eingedampften Extrakte (Kieselgel, 15 g/$CH_2Cl_2$) ergab 276 mg 1-[3-Oxo-pent-4-en-1-yl]-1-trimethylsilyloxy-2,2,6-trimethylcyclohexa-2,4-dien (47% Ausbeute), farbloses Oel, IR($CCl_4$): 1710, 1693, 1625, 1258, 1130, 905 $cm^{1}$).

0024358

## Patentansprüche

1. Substituierte Cyclohexadiene der Formel

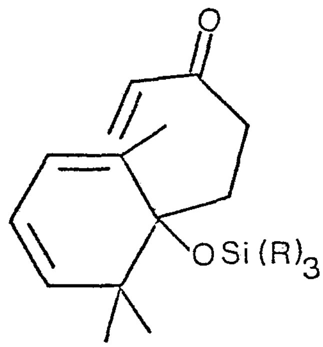

IV

worin R eine niedere Alkylgruppe, gegebenenfalls nieder Alkyl-substituiertes Phenyl oder Phenylniederalkyl bedeutet, worin die niederen Alkylgruppen 1-4 Kohlenstoffatome enthalten.

2. Substituierte Cyclohexadiene nach Anspruch 1, dadurch gekennzeichnet, dass R Methyl oder Aethyl bedeutet.

Europäisches Patentamt

# EUROPÄISCHER RECHERCHENBERICHT

0024358

Nummer der Anmeldung

EP 80105810.8

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | Keine Entgegenhaltungen. | | C 07 F 7/18<br>C 07 C 13/23<br>C 07 C 35/18//<br>A 61 K 7/46 |
| | | | **RECHERCHIERTE SACHGEBIETE (Int. Cl.³)** |
| | | | C 07 F<br>C 07 C 13/00<br>C 07 C 35/00<br>A 61 K |
| | | | **KATEGORIE DER GENANNTEN DOKUMENTE**<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 26-11-1980 | SCHÄFER |

EPA form 1503.1 06.78